# FASCICULE DE BREVET EUROPEEN

(11) **EP 1 138 879 B1**
(45) Date de publication et mention de la délivrance du brevet: **19.10.2005**
(21) Numéro de dépôt: 01400176.2
(22) Date de dépôt: 22.01.2001
(51) Int. Cl.: F01D 5/30

(54) **Disque rotorique de turbine équipé d'ailettes à pied de sapin et procédé de montage d'une ailette sur un disque**
Turbinenrotorscheibe und Schaufelbefestigung mittels Tannenbaumfuss und Montageverfahren
Turbine blade rotor disc assembly with fir-tree turbine blade root and assembly method therefore

(30) Priorité: 30.03.2000 FR 0004078
(43) Date de publication de la demande: 04.10.2001
(73) Titulaire: ALSTOM Power N.V., 1101 CS Amsterdam (NL)
(72) Inventeur: Tempere, Robert, 93600 Aulnay sous Bois (FR)
(74) Mandataire: Domenego, Bertrand

(56) Documents cités:
- EP-A- 0 597 586
- DE-A- 19 823 157
- US-A- 4 265 595
- US-A- 5 236 309

## Description

La présente invention concerne un disque rotorique de turbine équipé d'ailettes à pied de sapin.

Plus particulièrement, l'invention porte sur un moyen permettant aux ailettes à pied de sapin d'être bien solidarisées du disque sur lequel elles sont montées, même en faible vitesse de rotation. Le document US-A-5 236 309 de telles ailettes munies d'une barrette sur laquelle sont disposées des rondelles formant ressort.

En effet, avec ce mode de fixation où la base des ailettes est dite "en pied de sapin" et vient se loger dans une encoche complémentaire du disque rotorique sur lequel les ailettes sont montées, il faut une certaine vitesse de rotation pour que, grâce à la force centrifuge, aucun mouvement relatif ne soit possible. L'appui correct des dents du pied de sapin dans les rainures correspondantes d'une encoche du disque n'est en effet pas suffisant aux basses vitesses, aussi il est connu, pour remédier à cet inconvénient, de prévoir dans la face d'extrémité du pied de sapin quelques lamages, généralement trois, servant de logement pour y placer des ressorts.

Chaque ressort, constitué d'une simple lame bombée, est placé dans un lamage : les extrémités dans le lamage et la partie bombée tournée vers l'extérieur, c'est-à-dire contre le fond de l'encoche correspondante du disque rotorique. Bien entendu, la profondeur du lamage est inférieure à la flèche du ressort bombé.

Lors du montage des ailettes sur le disque, on place les ressorts dans leur lamage et le pied de sapin de chaque ailette est introduit dans l'encoche correspondante du disque, donc simultanément avec ses trois ressorts. Cette opération est très délicate et il survient fréquemment des incidents tels que : ressorts cassés et grippage.

La présente invention a pour but de proposer un disque rotorique de turbine équipé d'ailettes à pied de sapin, dans lequel le placage des dents des pieds de sapin des ailettes, contre les encoches complémentaires faites dans les encoches du disque, est assuré par des ressorts mais ne présentant pas, au montage, les inconvénients de l'art antérieur cité ci-dessus.

L'invention a ainsi pour objet un disque rotorique de turbine équipé d'ailettes à pied de sapin, ledit disque comportant des encoches complémentaires dans chacune desquelles est introduit le pied de sapin d'une ailette, caractérisé en ce qu'au moins une barrette dont l'une des faces comporte un lamage, dans lequel est emprisonnée une lame de ressort, est disposée dans le fond de chaque encoche du disque entre ce fond et la face d'extrémité du pied de sapin de l'ailette correspondante.

Avantageusement, trois dites barrettes sont disposées entre le fond d'une encoche du disque et la face d'extrémité du pied de sapin de l'ailette correspondante.

Selon une autre caractéristique, l'extrémité du pied de sapin des ailettes comporte une talonnette contre laquelle vient buter une dite barrette.

L'invention a aussi pour objet un procédé pour le montage d'une ailette à pied de sapin sur un disque rotorique de turbine conforme à l'invention, caractérisé par la suite des opérations suivantes :
a. Le disque rotorique est placé avec son axe en position horizontale.
b. L'encoche du disque, dans laquelle le pied de sapin de l'ailette à monter doit être introduit, est placée en position basse.
c. L'ailette est mise en place en introduisant par coulissement le pied de sapin dans l'encoche du disque.
d. La ou les barrettes, équipées chacune de leur lame de ressort, sont ensuite introduites dans l'espace libre entre le fond de l'encoche du disque et l'extrémité du pied de sapin de l'ailette, jusqu'à mise en butée de la première barrette introduite contre ladite talonnette du pied de sapin de l'ailette.

On va maintenant donner la description d'un exemple de mise en oeuvre de l'invention en se reportant au dessin annexé dans lequel :
La fig. 1 montre une vue partielle d'un disque rotorique de turbine à vapeur selon l'invention vu dans le sens de la flèche repérée I sur la fig. 2.
La fig. 2 est une vue en coupe selon II-II de la fig. 1.
La fig. 3 est un agrandissement de la partie III de la fig. 2.
Les fig. 4 et 5 montrent respectivement, en vue de dessus et en coupe selon V-V de la fig. 4, une barrette servant au montage d'une ailette sur le disque rotorique.
La fig. 6 montre schématiquement et partiellement un rotor de turbine dans lequel on voit un disque avec une ailette dans la position de montage.
La fig. 7 montre partiellement un disque avec une encoche en position basse.
La fig. 8 est une vue selon la flèche VIII de la fig. 7.
La fig. 9 montre une ailette en position de montage dans l'encoche visible fig. 7 et 8 du disque rotorique.
La fig. 10 montre l'ailette de la fig. 9 vue selon la flèche X.
Les fig. 11 et 12 sont similaires aux fig. 7 et 8 mais après le montage d'une ailette.
La fig. 13 est un agrandissement du détail XIII de la fig. 11.
La fig. 14 est un agrandissement du détail XIV de la fig. 11.
La fig. 15 montre un outil utilisé pour introduire les barrettes dans leur emplacement.

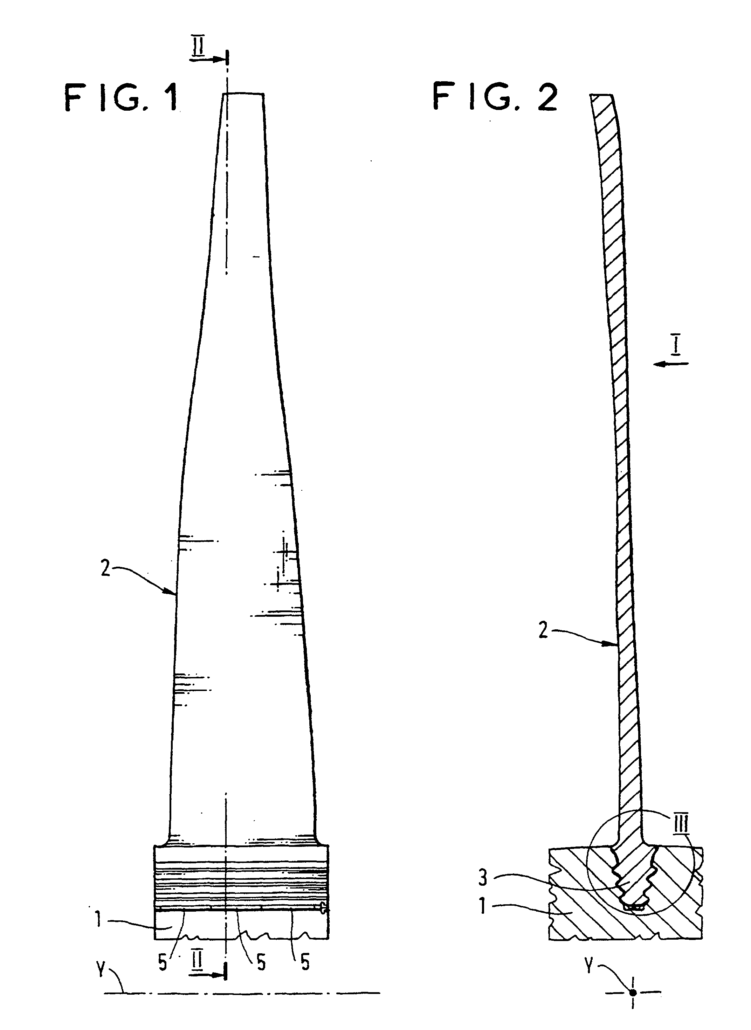

En se reportant aux fig. 1 à 3, on voit très partiellement un disque rotorique 1 de turbine à vapeur sur lequel est montée une ailette 2.

L'ailette est dite "à pied de sapin". Il s'agit de la base 3 de l'ailette dont la forme générale, vue selon une section perpendiculaire à l'axe Y du disque rotorique, ressemble à un sapin. Ce pied de sapin 3 a pour but de solidariser l'ailette 2 avec le disque 1. Le pied de sapin 3 est introduit dans une encoche 4 correspondante du disque 1.

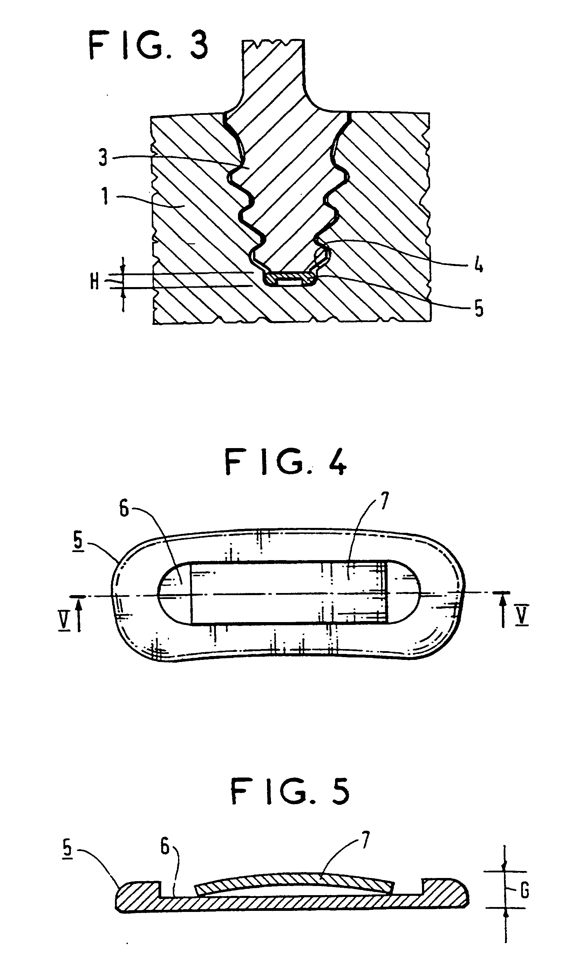

Conformément à l'invention, et comme on le voit sur le détail III représenté fig. 3 en vue agrandie, une barrette 5 est disposée entre le fond de l'encoche 4 et l'extrémité du pied de sapin 3 de l'ailette 2. En pratique, trois de ces barrettes sont introduites successivement les unes derrière les autres : elles sont juste figurées fig. 1.

Chaque barrette est équipée d'une lame de ressort, non visible sur ces fig. 1 à 3, mais une barrette vue isolément est représentée fig. 4 et 5. Comme on le voit sur ces figures, la barrette 5 comporte un lamage 6 dans lequel est placée, emprisonnée, une lame de ressort 7.

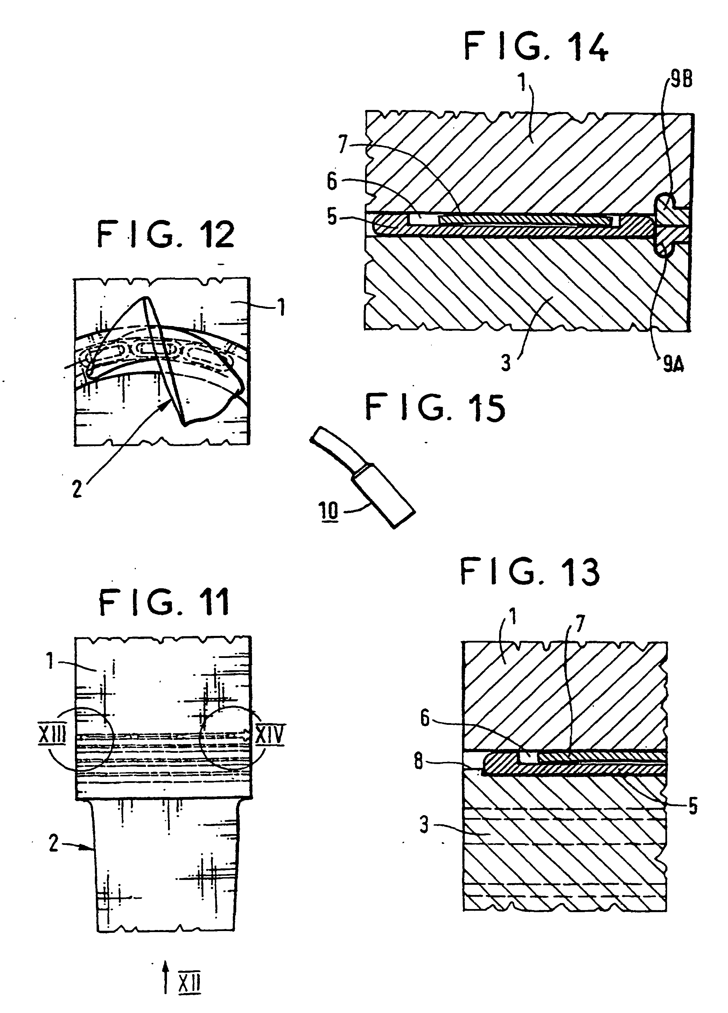

Sur les fig. 11 et 12, on voit l'ailette 2 montée sur le disque 1. La fig. 11 correspond à la fig. 1 mais le disque ayant été tourné de façon à placer l'ailette 2 vers le bas et la fig. 12 est une vue selon la flèche XII de la fig. 11.

Sur le détail repéré XIII fig. 11 et montré en vue agrandie fig. 13, on voit que l'extrémité du pied de sapin 3 de l'ailette 2 comporte une talonnette 8 contre laquelle vient buter la première barrette 5 introduite.

Le détail XIV de la fig. 11, montré en vue agrandie fig. 14, montre l'autre face du disque 1 où la série d'ailettes 2, toutes montées de la même façon qui vient d'être expliquée, est arrêtée latéralement d'une façon connue par un jonc en deux parties 9A, 9B, et plusieurs segments ensuite soudés.

Comme on le voit sur le détail représenté fig. 3, l'espace libre entre l'extrémité du pied de sapin 3 et le fond de l'encoche 4 dans le disque 1 a une hauteur H.

Sur la fig. 5 montrant la barrette 5 en vue isolée avec sa lame de ressort 7, qui est une simple lame bombée, la hauteur totale de la barrette avec son ressort au repos est égale à G et bien évidemment, afin que le ressort joue son rôle de plaquer les dents des pieds de sapin des ailettes contre les rainures des encoches du disque, la hauteur G est supérieure à H.

Comme le montre la fig. 5, les extrémités de la lame de ressort 7 sont au fond du lamage 6 et la partie bombée, au centre, est, lorsque la plaquette est introduite entre le pied de sapin et le fond de l'encoche, en appui contre le fond de l'encoche 4 du disque rotorique 1, tandis que la face plane de la barrette 5 est en appui contre l'extrémité du pied de sapin 3 (voir fig. 3, 13 et 14).

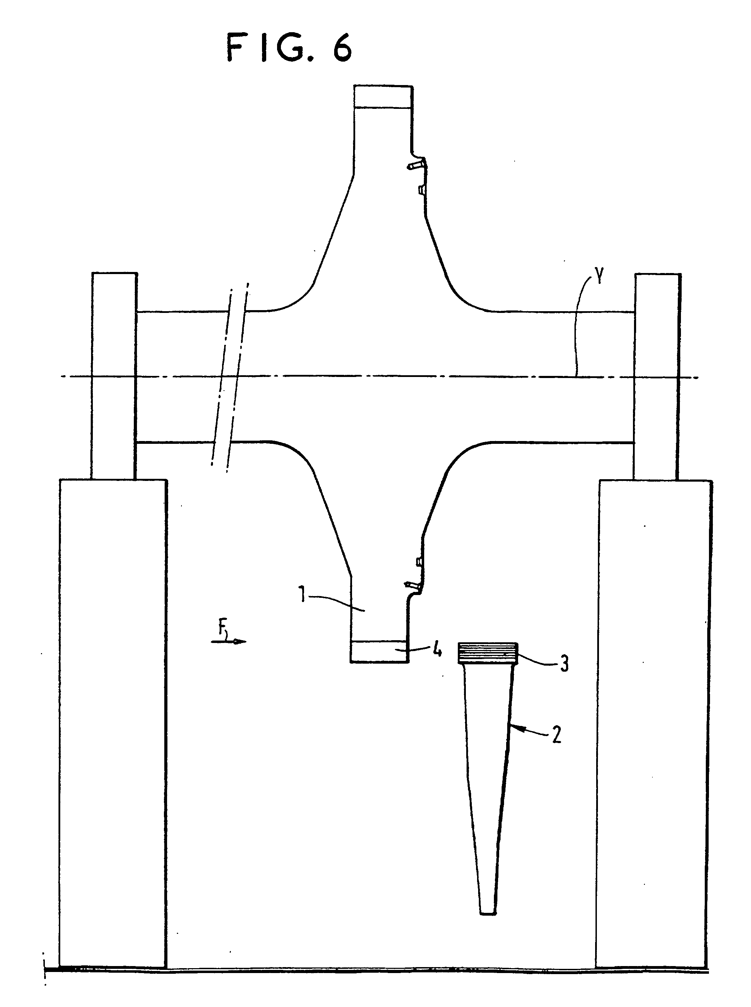

Les fig. 6 à 10 permettent de montrer le montage d'une ailette 2 sur le disque rotorique 1.

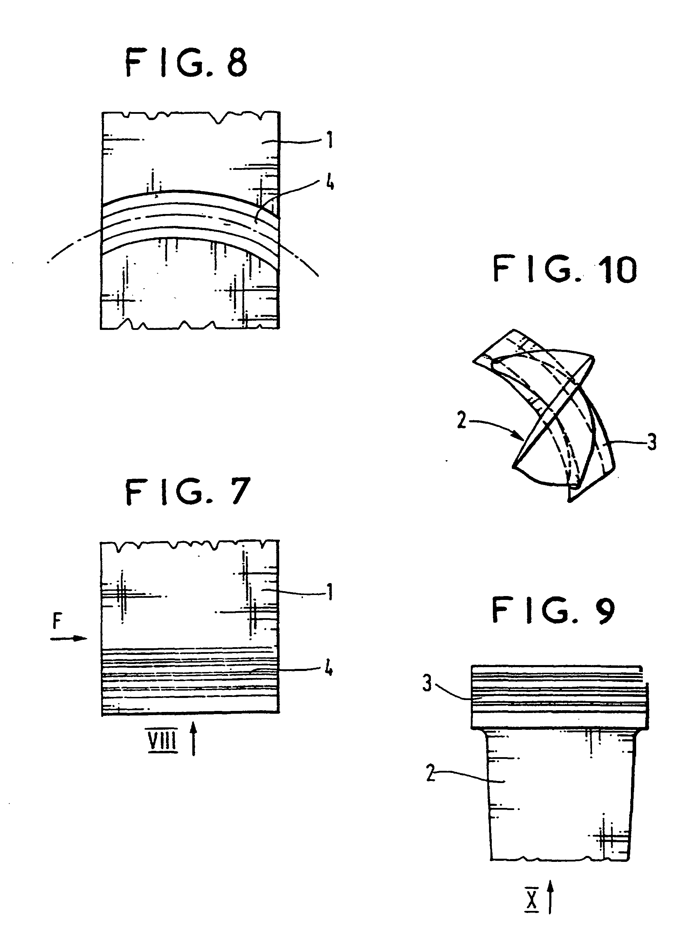

Comme le montre la fig. 6, la turbine, avec son disque 1, est placée avec son axe Y en position horizontale et l'ailette 2 à monter est introduite librement par son pied de sapin 3 dans une encoche 4 du disque située en position basse. C'est ce que l'on voit également fig. 7 et 9. Le sens de montage de l'ailette sur le disque se fait par la face de sortie de la vapeur sachant que le sens de la vapeur est représenté par la flèche F.

Les fig. 8 et 10 permettent de voir qu'en vue selon les flèches VIII et X des fig. 7 et 9, le profil de l'encoche 4 et, bien sûr, du pied de sapin 3 est incurvé.

Compte tenu de l'espace libre H (fig. 3) entre le fond de l'encoche 4 et l'extrémité du pied de sapin 3, celui-ci coulisse librement dans l'encoche 4 sans risque de grippage des dents du pied de sapin ni des rainures de l'encoche, l'état de surface de ces dents et rainures étant soigné.

Ensuite, la première barrette 5 est introduite dans l'espace libre entre l'extrémité du pied de sapin 3 et le fond de l'encoche 4 à l'aide d'un outil 10 représenté fig. 15. Il suffit de venir frapper à l'arrière de l'outil dans le sens de la flèche F₁ avec un maillet jusqu'à appui de la première barrette contre la talonnette 8 du pied de sapin 3 (fig. 13). On introduit de la même façon les deux autres barrettes jusqu'à appui contre la barrette précédente. L'on ferme ensuite de la manière habituelle par les joncs 9A, 9B (fig. 14).

## Revendications

1. Disque rotorique (1) de turbine équipé d'ailettes (2) à pied de sapin (3), ledit disque (1) comportant des encoches (4) complémentaires dans chacune desquelles est introduit le pied de sapin (3) d'une ailette (2), comportant ou moins une barrette (5) dont l'une des faces comporte un lamage (6), dans lequel est emprisonnée une lame de ressort (7), ladite lame de ressort (7) étant une lame bombée, les extrémités de la lame étant en appui dans ledit lamage (6) et la partie centrale bombée étant en appui contre le fond de ladite encoche du disque rotorique, la face opposée de la barrette étant en appui contre l'extrémité du pied de sapin de l'ailette.

2. Disque rotorique selon la revendication 1, **caractérisé en ce que** trois dites barrettes (5) sont disposées entre le fond d'une encoche du disque et la face d'extrémité du pied de sapin de l'ailette correspondante.

3. Disque rotorique selon l'une des revendications 1 ou 2, **caractérisé en ce que** l'extrémité du pied de sapin des ailettes comporte une talonnette (8) contre laquelle vient buter une dite barrette (5).

4. Procédé de montage d'une ailette à pied de sapin sur un disque rotorique de turbine conforme à l'une des revendications 1 à 3, **caractérisé par** la suite des opérations suivantes :
a. Le disque rotorique (1) est placé avec son axe (Y) en position horizontale.
b. L'encoche (4) du disque, dans laquelle le pied de sapin (3) de l'ailette à monter doit être introduit, est placée en position basse.
c. L'ailette est mise en place en introduisant par coulissement le pied de sapin (3) dans l'encoche (4) du disque.
d. La ou les barrettes (5), équipées chacune de leur lame de ressort (7), sont ensuite introduites dans l'espace libre entre le fond de l'encoche du disque et l'extrémité du pied de sapin de l'ailette, jusqu'à mise en butée de la première barrette introduite contre ladite talonnette (8) du pied de sapin de l'ailette.

5. Procédé de montage selon la revendication 4, **caractérisé en ce que** lesdites barrettes sont introduites côté sortie de la vapeur et la première barrette (5) introduite venant en butée contre ladite talonnette (8) située côté entrée de la vapeur.

## Patentansprüche

1. Turbinenrotorscheibe (1), die mit Schaufeln (2) mit Tannenbaumfuß (3) versehen ist, wobei die Scheibe (1) komplementäre Aussparungen (4) aufweist, in welche jeweils der Tannenbaumfuß (3) einer Schaufel (2) eingeführt wird, mit zumindest einem Steg (5), dessen eine Seite eine Vertiefung (6) aufweist, in welcher ein Federblatt (7) eingeschlossen ist, wobei das Federblatt (7) ein gewölbtes Blatt ist, wobei die Enden des Blattes in der Vertiefung (6) aufliegen und der mittlere gewölbte Bereich in Auflage auf dem Boden der Aussparung der Rotorscheibe ist, wobei sich die gegenüber liegende Seite des Steges in Auflage auf dem Ende des Tannenbaumfußes der Schaufel befindet.

2. Rotorscheibe gemäß Anspruch 1, **dadurch gekennzeichnet, dass** drei Stege (5) zwischen dem Boden einer Aussparung der Scheibe und der Endfläche des Tannenbaumfußes der entsprechenden Schaufel angeordnet sind.

3. Rotorscheibe gemäß einem der Ansprüche 1 oder 2, **dadurch gekennzeichnet, dass** das Ende des Tannenbaumfußes der Schaufeln einen kleinen Ansatz (8) aufweist, gegen den ein Steg (5) in Anschlag kommt.

4. Verfahren zur Montage einer Schaufel mit Tannenbaumfuß an einer Turbinenrotorscheibe gemäß einem der Ansprüche 1 bis 3, **gekennzeichnet durch** die Abfolge der folgenden Schritte:
a. Die Rotorscheibe (1) wird mit ihrer Achse (Y) in horizontaler Position angeordnet.
b. Die Aussparung (4) der Scheibe, in welche der Tannenbaumfuß (3) der zu montierenden Schaufel eingefügt werden soll, wird in unterer Position angeordnet.
c. Die Schaufel wird angeordnet, indem der Tannenbaumfuß (3) **durch** Gleiten in die Aussparung (4) der Scheibe eingeführt wird.
d. Der oder die Stege (5), die jeweils mit ihrem Federblatt (7) versehen sind, werden dann in den freien Raum zwischen dem Boden der Aussparung der Scheibe und dem Ende des Tannenbaumfußes der Schaufel soweit eingeschoben, bis dass der erste eingeschobene Steg gegen den kleinen Ansatz (8) des Tannenbaumfußes der Schaufel anschlägt.

5. Montageverfahren gemäß Anspruch 4, **dadurch gekennzeichnet, dass** die Stege auf der Austrittsseite des Dampfes eingeführt werden und der erste eingeführte Steg (5) in Anschlag gegen den auf der Dampfeintrittsseite angeordneten kleinen Ansatz (8) kommt.

## Claims

1. A turbine rotor disk (1) fitted with blades (2) having Christmastree-shaped roots (3), said disk (1) having complementary slots (4) each receiving the Christmastree-shaped root (3) of a blade, comprising at least one stick (5), one face of which having a spot facing (6) holding a spring strip (7) captive, wherein said spring strip (7) is a curved strip with the ends of the strip bearing in said spot facing (6) and with the curved central portion thereof bearing against the bottom of said slot of the rotor disk, the opposite face of the stick bearing against the end of the Christmastree-shaped root of the blade.

2. A rotor disk (1) according to claim 1, wherein three of said sticks (5) are disposed between the bottom of a slot in the disk and the end face of the root of the corresponding blade.

3. A rotor disk according to anyone of claims 1 or 2, wherein the end of the Christmastree-shaped root of the blades has a rim (8) against which one of said sticks (5) comes into abutment.

4. A method of assembling a blade having a Christmastree-shaped root to a turbine rotor disk according to anyone of claims 1 to 3, the method comprising the following sequence of operations:
a) the rotor disk (1) is placed so that its axis (Y) is in a horizontal position;
b) the slot (4) of the disk in which the Christmastree-shaped blade root (3) to be mounted is placed in the bottom position;
c) the blade is installed by slidably inserting the Christmastree-shaped root (3) of the blade in the slot (4)of the disk; and
d) the stick(s) (5) each fitted with their respective spring strip (7) are then inserted into the empty space between the bottom of the slot in the disk and the end of the blade Christmastree-shaped root until the first-inserted stick comes into abutment against said rim (8) on the Christmastree-shaped root of the blade.

5. An assembly method according to claim 4, wherein said sticks are inserted from the steam outlet side and the first stick (5) to be inserted coming into abutment against said rim (8) which is situated at the steam inlet side.
